# EUROPEAN PATENT APPLICATION

(11) **EP 1 808 157 A1**
(43) Date of publication of application: **18.07.2007**
(21) Application number: 07000697.8
(22) Date of filing: 15.01.2007
(51) Int. Cl.: A61K 8/06, A61K 8/34, A61K 8/36, A61K 8/41, A61K 8/58, A61K 8/92, A61Q 5/12

(54) **Hair cosmetic compositions and process for producing the same**

(30) Priority: 13.01.2006 JP 2006006721
(71) Applicant: Kao Corporation, Chuo-ku Tokyo (JP)
(72) Inventor: Kaharu, Takeshi Kao Corporation, Tokyo (JP)
(74) Representative: HOFFMANN EITLE

(57) **Abstract**

There are provided a hair cosmetic composition capable of imparting a light moistened feel, a good smoothness and a good manageability to hair after drying which is free from frictional feel upon rinsing and exhibit an effect of mending damaged portions of the hair, and a process for producing the composition. The hair cosmetic composition of the present invention includes a gel emulsion containing at least one fatty alcohol, a cationic surfactant and an aqueous carrier, and (A) an oil agent phase containing a vegetable oil and a volatile silicone, wherein the oil agent phase (A) is present in the form of oil droplet particles in the gel emulsion.

## Description

### FIELD OF THE INVENTION

The present invention relates to hair cosmetic compositions in which a vegetable oil and a silicone oil are stably suspended, and a process for producing the composition.

### BACKGROUND OF THE INVENTION

Hair cosmetic compositions have been required to impart good softness and smoothness during wetting through drying as well as good moistened feel and manageability after drying to hair. In general, after shampooing, a hair cosmetic composition such as a hair rinse, a hair conditioner and a hair treatment has been used to improve inconveniences occurring upon styling such as dry and rough feel, poor smoothness and poor comb-passing of hair. As such a hair cosmetic composition, there have been proposed various compositions containing a cationic surfactant as a main component together with a conditioning component such as a silicone oil and a hydrocarbon-based oil.

For example, JP 2-172906A discloses a hair cosmetic composition containing a partially crosslinked silicone, a liquid oil such as a silicone oil and an ester oil, and a volatile oil agent which composition is excellent in adhesion to hair and conditioning effect. JP 5-262624A discloses a hair cosmetic composition in which a cationic surfactant is used in combination with a branched fatty ester and silicones in order to enhance touch feel of hair and prevent dry and rough feel thereof. JP 2000-159637A discloses a hair cosmetic composition in which a cationic surfactant is used in combination with a silicone oil, a diglycerol carboxylic acid ester and a condensed product of dicarboxylic acid in order to enhance a conditioning effect and a moistened feel. Further, JP 2003-327513A discloses a hair cosmetic composition in which an animal or vegetable oil or a hydrocarbon oil is used in combination with an ether-type cationic surfactant in order to impart a good smoothness, a good softness and a good moistened feel to hair upon wetting and even after drying.

### SUMMARY OF THE INVENTION

However, with the recent increasing concern for hair styling, hair has been frequently subjected to chemical treatments such as bleach treatment and permanent wave treatment as well as drying by a dryer and ironing. Therefore, there arises a strong tendency that the hair suffers from damage to hair cuticle and change in an internal structure of the hair which leads to deterioration in finger passing, luster and softness thereof. As a result, there tends to occur remarkable damage to hair such as damaged hair tip portions as well as dry and rough feel, spread and poor touch feel of the hair.

Thus, there is a demand for a hair cosmetic composition capable of imparting a smooth, wet and moistened finish feel even to excessively damaged hair after drying.

The present invention relates to a hair cosmetic composition capable of imparting a soft and moistened finish feel even to damaged hair and keeping the hair in a healthy condition, and exhibiting a good feeling upon use.

The inventors have found that when a separately prepared mixture of a vegetable oil and a volatile silicone is added into a gel emulsion containing an oil component and water, individual oil droplet particles of the vegetable oil are formed in the gel emulsion, and the resultant composition can effectively exhibit a good vegetable oil-derived conditioning effect.

Thus, the present invention relates to a hair cosmetic composition including a gel emulsion containing at least one fatty alcohol, a cationic surfactant and an aqueous carrier, and (A) an oil agent phase containing a vegetable oil and a volatile silicone, wherein the oil agent phase (A) is present in the form of oil droplet particles in the gel emulsion. Further, the present invention relates to a process for producing a hair cosmetic composition in which oil droplet particles containing a vegetable oil are present in the form of individual particles in a gel emulsion.

### EFFECT OF THE INVENTION

The hair cosmetic composition of the present invention can impart a softness and a moistened feel even to damaged hair and keep the hair in a healthy condition.

### DETAILED DESCRIPTION OF THE INVENTION

The gel emulsion used in the present invention contains at least one fatty alcohol, a cationic surfactant and an aqueous carrier. The fatty alcohol used herein includes fatty alcohols containing a linear or branched alkyl or alkenyl group having 8 to 30 carbon atoms. Among these fatty alcohols, especially preferred are fatty alcohols containing a linear or branched alkyl or alkenyl group having 10 to 26 carbon atoms. Specific examples of the fatty alcohol include cetyl alcohol, stearyl alcohol, arachyl alcohol, behenyl alcohol, caranabyl alcohol and ceryl alcohol. Among these fatty alcohols, preferred are cetyl alcohol, stearyl alcohol and behenyl alcohol.

These fatty alcohols may be used in combination of any two or more thereof. The content of the fatty alcohol in the gel emulsion is preferably from 0.5 to 15% by mass on the basis of a whole amount of the resultant composition, and the content of the fatty alcohol is more preferably from 1 to 10% by mass and still more preferably from 2 to 7% by mass in view of feeling upon use, a texture (viscosity) as a cosmetic and a storage stability.

The gel emulsion may also contain, in addition to the fatty alcohol, other oil agents and fats and oils. Examples of the other oil agents and fats and oils include hydrocarbons, waxes, higher fatty acids, esters and ethers. Specific examples of the other oil agents and fats and oils include hydrocarbons such as squarane, squalene, liquid paraffins, liquid isoparaffins and cycloparaffins; waxes such as beeswax, lanolin and carnauba wax; higher fatty acids such as myristic acid, palmitic acid, stearic acid, behenic acid, oleic acid, coconut oil fatty acid, isostearic acid and isopalmitic acid; esters such as isopropyl palmitate, isopropyl myristate, octyldodecyl myristate, hexyl laurate, stearyl stearate, cetyl lactate; propylene glycol monostearate, oleyl oleate, hexadecyl 2-ethylhexanoate, isononyl isononanoate and isotridecyl isononanoate; and ethers such as isostearyl glyceryl ether.

Further, in order to enhance an effect of externally mending hair fibers, the hair cosmetic composition may also contain an amphoteric amide lipid. Examples of the amphoteric amide lipid include those compounds selected from diamide compounds represented by the following general formula (II), and ceramides:

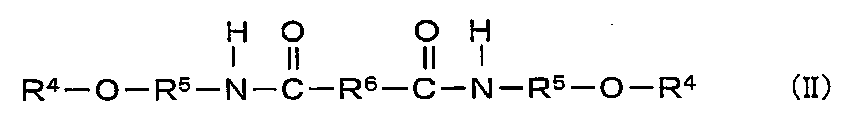

In the general formula (II), R⁴ is an unsubstituted alkyl group having 1 to 12 carbon atoms, or an alkyl group having 2 to 12 carbon atoms which is substituted with one or two hydroxyl groups, one alkoxy group having 1 to 6 carbon atoms, or both one hydroxyl group and one alkoxy group having 1 to 6 carbon atoms; R⁵ is a linear or branched alkylene group having 2 to 5 carbon atoms and preferably 2 to 3 carbon atoms; and R⁶ is a linear or branched alkylene group having 11 to 22 carbon atoms, and preferably an alkenylene group having 1 to 4 double bonds.

Specific examples of the diamide compounds include those represented by the following formulae:

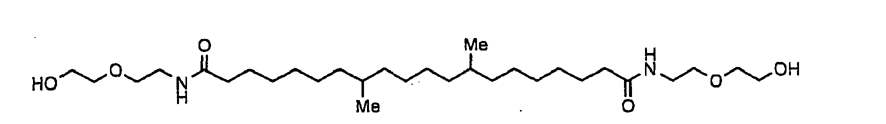

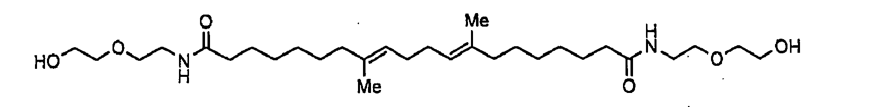

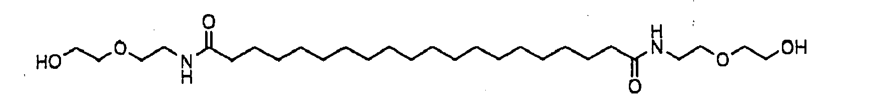

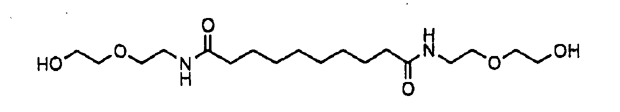

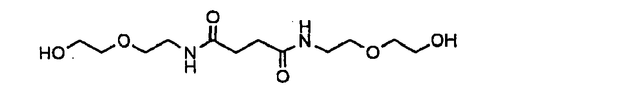

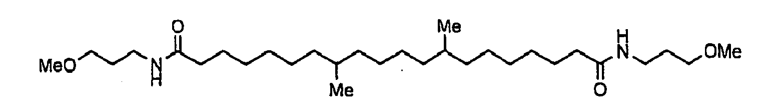

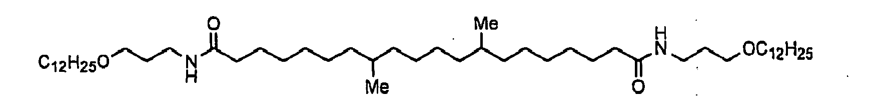

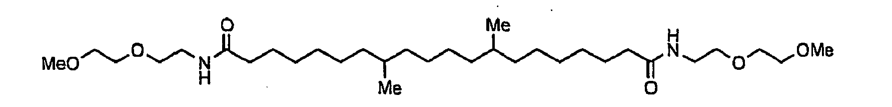

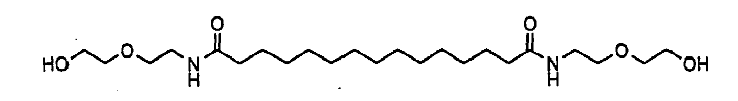

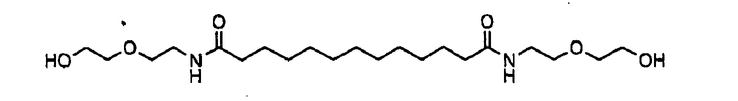

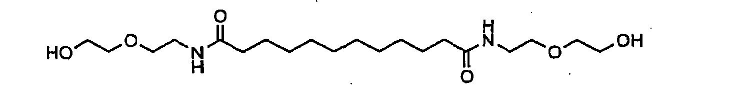

Examples of the ceramides include N-acylated sphingosines, N-acylated phytosphingosines and N-acylated dihydrosphingosines. The fatty acid residue used for acylation of sphingosine, phytosphingosine and dihydrosphingosine have a linear or branched chain having 8 to 22 carbon atoms, and 1 to 5 hydrogen atoms in the fatty acid residue may be respectively substituted with a hydroxyl group. Specific examples of the ceramides include ceramide 1, ceramide 2, ceramide 3, ceramide 1A, ceramide 6II, hydroxycaproyl phytosphingosine (for example, porcine or human ceramides described in Fig. 2 of "J. Lipid Res.", 24, 759 (1983) and Fig. 4 of "J. Lipid Res.", 35, 2069 (1994)) and natural ceramides such as N-alkyl compounds (for example, N-methyl compounds) of these ceramides; and synthetic pseudo-ceramides as described in JP 11-209248A and JP 01-042934B and synthetic pseudo-ceramides represented by the following formulae:

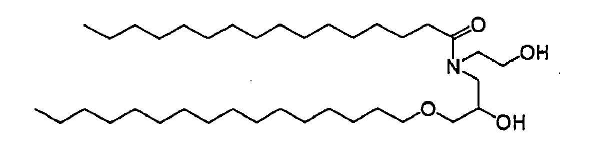

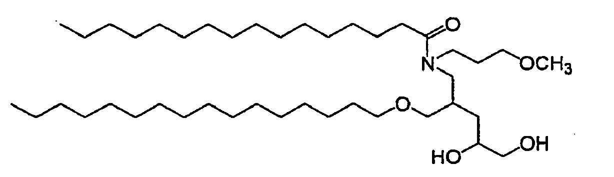

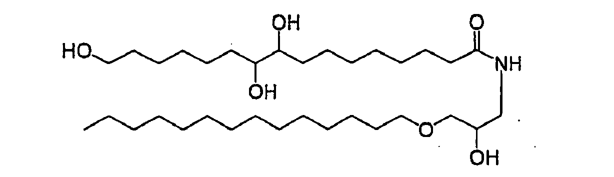

The content of the amphoteric amide lipid in the hair cosmetic composition is from about 0.01 to about 5% by mass, and the content of the amphoteric amide lipid is preferably from 0.05 to 2% by mass and more preferably from 0.1 to 1% by mass in view of a sufficient effect of externally mending hair fibers.

Examples of the cationic surfactant suitably used in the present invention include the following compounds (i) to (vi).
(i) Alkyltrimethylammonium Salt

   R⁷⁻N⁺(CH₃)₃X⁻

   wherein R⁷ is an alkyl group having 12 to 22 carbon atoms; and X⁻ is a halogen ion such as chloride ion and bromide ion.
   Specific examples of the compound (i) include cetyltrimethylammonium chloride, stearyltrimethylammonium chloride and behenyltrimethylammonium chloride.
(ii) Alkoxytrimethylammonium Salt

   R⁸-O-R⁹-N(CH₃)₃X⁻

   wherein R⁸ is an alkyl group having 12 to 22 carbon atoms; R⁹ is an ethylene group, a propylene group or a hydroxypropylene group; and X' is the same as defined above.
   Specific examples of the compound (ii) include stearoxypropyltrimethylammonium chloride, stearoxyethyltrimethylammonium chloride and stearoxyhydroxypropyltrimethylammonium chloride.
(iii) Dialkyldimethylammonium Salt

   R¹⁰₂N⁺(CH₃)₂X⁻

   wherein R¹⁰ is an alkyl group having 12 to 22 carbon atoms or a benzyl group; and X⁻ is the same as defined above.
   Specific examples of the compound (iii) include distearyldimethylammonium chloride.
(iv) Alkyldimethylamine and Salt thereof

   R¹¹-N(CH₃)₂

   wherein R¹¹ is an alkyl group having 12 to 22 carbon atoms.
   Specific examples of the compound (iv) include organic acid salts of behenyldimethylamine and stearyldimethylamine.
(v) Alkoxydimethylamine and Salt thereof

   R¹²-O-R¹³-N(CH₃)₂

   wherein R¹² is an alkyl group having 12 to 22 carbon atoms; and R¹³ is an ethylene group, a propylene group or a hydroxypropylene group.
   Specific examples of the compound (v) include organic acid salts of stearoxypropyldimethylamine and stearoxyhydroxypropyldimethylamine.
(vi) Alkylamidodimethylamine and Salt thereof

   R¹⁴-C(=O)NH-R¹⁵-N(CH₃)₂
wherein R¹⁴ is an alkyl group having 11 to 21 carbon atoms; and R¹⁵ is an ethylene group or a propylene group.

Specific examples of the compound (vi) include organic acid salts of stearamidoproryl dimethylamine and behenamidoproryl dimethylamine.

Also, as the acid for neutralizing the cationic surfactant (iv) to (vi) mentioned above, there may be used inorganic acids and organic acids having 10 or less carbon atoms. Examples of the acid include those acids containing a short-chain alkyl group having 10 or less carbon atoms such as alkyl phosphoric acids, alkyl sulfonic acids and alkyl sulfuric acids; acidic amino acids such as L-glutamic acid, L-aspartic acid and pyroglutamic acid; aromatic acids such as benzoic acid and p-toluenesulfonic acid; hydroxy acids; and dicarboxylic acids. Specific examples of the hydroxy acids include monohydroxy carboxylic acids such as glycolic acid, lactic acid, glyceric acid, gluconic acid and pantothenic acid; hydroxy dicarboxylic acids such as malic acid and tartaric acid; and hydroxy tricarboxylic acids such as citric acid. Specific examples of the dicarboxylic acids include oxalic acid, malonic acid, maleic acid, succinic acid and glutaric acid. Among these acids, especially preferred are hydroxy acids, dicarboxylic acids, L-glutamic acid, L-aspartic acid and pyroglutamic acid, and more preferred are glycolic acid, lactic acid, malic acid, L-glutamic acid, L-aspartic acid and pyroglutamic acid.

In the present invention, the above acid is preferably used in an amount of 1 to 10 mol, more preferably 1.5 to 10 mol and still more preferably 1.5 to 6 mol on the basis of an amine equivalent of the cationic surfactant.

In particular, when the hydroxy acid or the dicarboxylic acid, more specifically glycolic acid, lactic acid or malic acid, is used in an excessive amount relative to the cationic surfactant, it is expected that the obtained composition exhibits the effect of mending or preventing damaged hair (voids inside of hair fibers).

The content of the acid in the hair cosmetic composition is preferably from 0.1 to 10% by mass and more preferably from 0.1 to 5% by mass.

The above cationic surfactants may be used in combination of any two or more thereof. The content of the cationic surfactant in the hair cosmetic composition is preferably from 0.1 to 10% by mass, more preferably from 0.1 to 5% by mass and still more preferably from 0.3 to 3% by mass in view of imparting a good softness and a good smoothness to hair.

The aqueous carrier as a component of the gel emulsion may contain, in addition to purified water, a lower alcohol, etc. Examples of the lower alcohol include ethyl alcohol, propyl alcohol and butyl alcohol. These aqueous carriers may be used alone or in combination of any two or more thereof. The content of the aqueous carrier in the hair cosmetic composition of the present invention is preferably from 30 to 98% by mass, more preferably from 50 to 95% by mass and still more preferably from 75 to 95% by mass.

The gel emulsion may also contain an organic solvent, a polymer, etc. These components may be added to the gel emulsion in the form of a mixture with the oil phase or the aqueous carrier, or separately therefrom, according to requirements.

Examples of the organic solvent include aromatic alcohols such as benzyl alcohol, cinnamyl alcohol, phenethyl alcohol, p-anisyl alcohol, p-methylbenzyl alcohol, phenoxy ethanol and 2-benzyloxy ethanol; polyhydric alcohols such as ethylene glycol, propylene glycol, 1,3-butanediol and glycerol; N-alkyl pyrrolidones formed by bonding an alkyl group having 1 to 18 carbon atoms to a nitrogen atom of pyrrolidone such as N-methyl pyrrolidone, N-octyl pyrrolidone and N-lauryl pyrrolidone; alkylene carbonates containing an alkylene group having 2 to 4 carbon atoms such as ethylene carbonate and propylene carbonate; polypropylene glycols having a molecular weight of 200 to 5000, preferably 200 to 1000 and more preferably about 400; lactones such as γ-butylolactone, γ-caprolactone, γ-valerolactone, δ-valerolactone, δ-caprolactone and δ-heptanolactone; and cyclic ketones such as cyclopentanone, cyclohexanone, cycloheptanone and 4-methyl cycloheptanone. Among these organic solvents, especially preferred are aromatic alcohols, polyhydric alcohols, alkylene carbonates and polypropylene glycols.

These organic solvents may be used in combination of any two or more thereof. The content of the organic solvent in the hair cosmetic composition is preferably from 0.1 to 20.0% by mass, more preferably from 0.1 to 10.0% by mass and still more preferably from 0.1 to 7% by mass in view of improving a finish feel such as, for example, luster, manageability, suppleness and elasticity of hair.

The hair cosmetic composition of the present invention may further contain an additional surfactant such as a nonionic surfactant and an amphoteric surfactant. These surfactants may be added to the gel emulsion, or may be blended in the below-mentioned oil agent phase (A) or (B), if used in a small amount, according to requirements. Examples of the nonionic surfactant include polyoxyalkylene sorbitan fatty esters, polyoxyalkylene sorbitol fatty esters, polyoxyalkylene glycerol fatty esters, polyoxyalkylene fatty esters, polyoxyalkylene alkyl ethers, polyoxyalkylene alkyl phenyl ethers, polyoxyalkylene (hardened) castor oils, sucrose fatty esters, polyglycerol alkyl ethers, polyglycerol fatty esters, fatty acid alkanol amides and alkyl glycosides.

Among these nonionic surfactants, preferred are alkyl glycosides, polyoxyalkylene (C₈ to C₂₀) fatty esters, polyoxyethylene sorbitan fatty esters, polyoxyethylene hardened castor oils and fatty acid alkanol amides. The fatty acid alkanol amides are preferably those containing an acyl group having 8 to 18 carbon atoms and preferably 10 to 16 carbon atoms, and may be in the form of either a monoalkanol amide or a dialkanol amide. The fatty acid alkanol amides are more preferably those containing a hydroxyalkyl group having 2 to 3 carbon atoms. Specific examples of the fatty acid alkanol amides include oleic acid diethanol amide, palm kernel oil fatty acid diethanol amide, coconut oil fatty acid diethanol amide, lauric acid diethanol amide, polyoxyethylene coconut oil fatty acid monoethanol amide, coconut oil fatty acid monoethanol amide, lauric acid isopropanol amide and lauric acid monoethanol amide. Examples of the amphoteric surfactant include betaine-based surfactants.

The content of the surfactant including the cationic surfactant in the hair cosmetic composition is preferably from 0.05 to 20% by mass, more preferably from 0.1 to 10% by mass and still more preferably from 0.1 to 5% by mass.

As the polymer, there may be used cationic polymers, nonionic polymers and amphoteric polymers. Examples of the cationic polymer include cationized cellulose derivatives, cationized starches, cationized guar gum derivatives, diallyl dialkyl quaternary ammonium salt/acrylamide copolymers, quaternarized polyvinyl pyrrolidone derivatives and condensates of polyglycol and polyamine.

Specific examples of the cationic polymer include cationized celluloses having a molecular weight of about 100,000 to 3,000,000, cationized starches having a cationization degree of about 0.01 to 1, cationized guar gums having a cationization degree of about 0.01 to 1, diallyl dialkyl quaternary ammonium salt/acrylamide copolymers having a molecular weight of 30,000 to 2,000,000, quaternarized polyvinyl pyrrolidone derivatives having a molecular weight of 10,000 to 2,000,000 in which the content of a cationic nitrogen in a vinyl polymer unit thereof is from 1.8 to 2.4%, such as quaternarized products of polyvinyl pyrrolidone/dimethylaminoethyl methacrylate copolymers, condensates of polyglycol and polyamine which contain an alkyl group having 6 to 20 carbon atoms, adipic acid/dimethylaminohydroxypropyl diethylene triamine copolymers, cationic polymers described on page 14, right upper column, line 18 through page 33, left lower column, line 2 of JP 53-139734A and on page 8, right upper column, line 17 through page 10, right upper column, line 6 of JP 60-36407A, and alkyl acrylamide/acrylate/alkylaminoalkyl acrylamide/polyethylene glycol methacrylate copolymers. Examples of the commercially available cationic polymers include "LUBIQUAT FC370", "LUBIQUAT FC550", "LUBIQUAT FC905", "LUBIQUAT HM552" and "LUBIQUAT MonoCP" all available from BASF (vinyl imidazolium trichloride/vinyl pyrrolidone copolymers); "CELQUAT H-100" (viscosity: 1000 cP) and "CELQUAT L-200" (viscosity: 100 cP) both available from National Starch Corp. (hydroxyethyl cellulose/dimethyl diallyl ammonium chloride); "GAFQUAT 734", "GAFQUAT 755N" and "GAFQUAT 755" all available from GAF Corp. (vinyl pyrrolidone/quaternarized dimethylaminoethyl methacrylate copolymers); "LUBIFLEX" available from BASF; "Copolymer 845", "Copolymer 937" and "Copolymer 958" all available from GAF Corp. (polyvinyl pyrrolidone/alkylamino acrylate copolymers); "Copolymer VC-713" available from GAF Corp., (polyvinyl pyrrolidone/alkylamino acrylate/vinyl caprolactam copolymers); and "GAFQUAT HS-100" available from ISP Corp. (vinyl pyrrolidone/methacrylamide propyl trimethyl ammonium chloride copolymer).

Examples of the nonionic polymer include hydroxyethyl cellulose, polyethylene glycol, a highly-polymerized polyethylene glycol and polyvinyl pyrrolidone. Specific examples of the commercially available nonionic polymers include hydroxyethyl celluloses such as "SE 900", "SE 850", "SE 600", "SE 550" and "SE 400" all available from Daicel Co., Ltd.; highly-polymerized polyethylene glycols such as "Polyox", "WSRN-12K", "WSRN-60K" and "WSR-301" all available from Amerchol Corp., and "PEO-27", "PEO-18Z", "PEO-15Z" and "PEO-8Z" all available from Sumitomo Seika Co., Ltd.; polyvinyl pyrrolidones such as "LUBISCOL K90", "LUBISCOL K80" and "LUBISCOL K30" all available from BASF; and polyvinyl alcohols such as "GOSENOL NH-26", "GOSENOL NH-20", "GOSENOL AH-26" and "GOSENOL AH-22" all available from Nippon Gosei Kagaku Co., Ltd.

Examples of the amphoteric polymers include polymers or copolymers of carboxybetaine-type monomers such as "PLASIZE L401" available from Gooh Kagaku Co., Ltd., and "YUCAFORMER AM-75" and "YUCAFORMER AM-75S/SM" both available from Mitsubishi Chemical Corp.; and acrylic acid/diallyl quaternary ammonium salt/acrylamide copolymers such as "MERCOAT PLUS 3330" available from CALGON Corp., and "ANFORMER 28-4910" and "ANFORMER LV-71" both available from National Starch Corp.

The above polymers may be used alone or in combination of any two or more thereof. The content of the polymer in the hair cosmetic composition is preferably from 0.05 to 5% by mass and more preferably from 0.05 to 1% by mass, in particular, in view of an adequate stickiness and feeling upon use such as a styling property.

The gel emulsion preferably has a viscosity at 25°C of 7 x 10³ to 7 x10⁴ mPa·s. When the viscosity of the gel emulsion lies within the above-specified range, the below-mentioned oil droplet particles are free from unification thereof, thereby ensuring a good dispersibility in the gel emulsion and, therefore, resulting in facilitated production of the composition. Meanwhile, the viscosity of the gel emulsion is measured at 30°C by a B-type viscometer using a rotor No. 4 at 12 rpm or using a rotor T-C at 10 rpm.

In the hair cosmetic composition of the present invention, the oil agent phase (A) containing a vegetable oil and a volatile silicone is present in the form of oil droplet particles in the gel emulsion. Namely, in the hair cosmetic composition of the present invention, since the vegetable oil is present in the form of oil droplet particles in the gel emulsion without being dissolved therein, the vegetable oil can be efficiently applied to hair fibers. The oil droplet particles being present independently of the gel emulsion may be produced by uniformly mixing the vegetable oil and the volatile silicone with each other to prepare a uniformly mixed oil layer (A) and then adding the oil layer to the gel emulsion, followed by mixing the obtained mixture under stirring. Meanwhile, formation of the oil droplet particles may be confirmed by observing the mixture by an optical microscope.

Examples of the vegetable oil usable in the present invention include sunflower oil, avocado oil, tsubaki oil, camellia oil, macadamia nuts oil, olive oil, jojoba oil, corn oil, rape seed oil, sesame oil, castor oil, linseed oil and mink oil. These vegetable oils may be used alone or in combination of any two or more thereof.

The content of the vegetable oil in the oil agent phase (A) is preferably from 5 to 50% by mass. The vegetable oil being present in the above-specified range tends to be uniformly mixed with the silicone and further stably present in the form of oil droplet particles in the gel emulsion. The content of the vegetable oil in the hair cosmetic composition of the present invention is preferably from 0.01 to 5% by mass. In particular, the content of the vegetable oil in the composition is more preferably from 0.05 to 5% by mass and still more preferably from 0.05 to 3% by mass in view of exhibiting a sufficient effect of mending damaged hair as well as imparting a good softness, a good moistened feel and a good luster to the hair.

The volatile silicone used in the present invention includes linear or cyclic silicone oils. Examples of the chain-like and cyclic silicones include chain-like silicones represented by the following general formula (III) and cyclic silicones represented by the following general formula (IV), respectively: 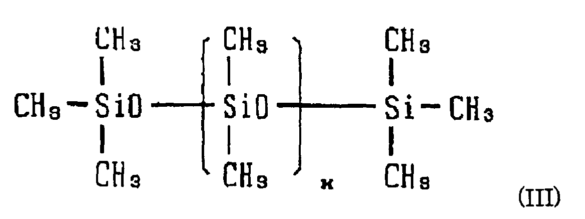 wherein x is an integer of 1 to 5; and 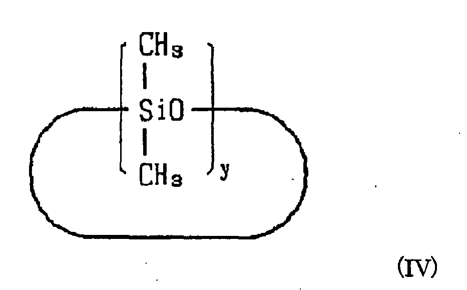 wherein y is an integer of 3 to 6.

Among these silicones, in view of forming a uniform mixture with the vegetable oil in the oil agent phase (A) and allowing the oil droplet particles to be stably present in the gel emulsion, preferred are cyclic silicones having 4 to 6 silicon atoms such as, in particular, octamethyl cyclotetrasiloxane and decamethyl cyclopentasiloxane, hexamethyl disiloxane, octamethyl trisiloxane, decamethyltetrasiloxane and dodecamethylpentasiloxane ; and most preferred is decamethyl cyclopentasiloxane as the cyclic silicone having 5 silicon atoms.

In the present invention, the mass ratio of the volatile silicone to the vegetable oil is preferably from 10:1 to 2:1 in view of formation of a uniform mixture of the volatile silicone and the vegetable oil in the oil agent phase (A) and a good stability of the oil droplet particles in the gel emulsion.

The content of the volatile silicone in the hair cosmetic composition of the present invention is preferably from 0.1 to 10% by mass, more preferably from 0.1 to 5% by mass and still more preferably from 0.2 to 3% by mass.

Also, the oil agent phase (A) preferably contains an amino-modified silicone represented by the following general formula (I):

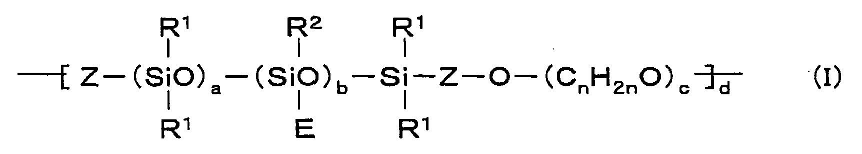

In the general formula (I), a plurality of the R¹ groups are each independently a hydrogen atom or a monovalent hydrocarbon group having 1 to 6 carbon atoms, preferably an alkyl group or a phenyl group, more preferably methyl or ethyl and most preferably methyl.

R² is either R¹ or E ; and E is a group represented by the formula: -R³-W wherein R³ is a direct bond or a divalent hydrocarbon group having 1 to 20 carbon atoms, preferably a linear or branched alkylene group having 1 to 6 carbon atoms such as a methylene group, an ethylene group, a trimethylene group, a propylene group or a tetramethylene group, and more preferably a trimethylene group or a propylene group. W is a primary to tertiary amino-containing group or an ammonium-containing group and preferably an amino-containing group or an ammonium-containing group represented by the following general formulae (V) and (VI):

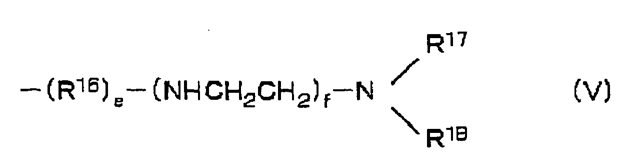

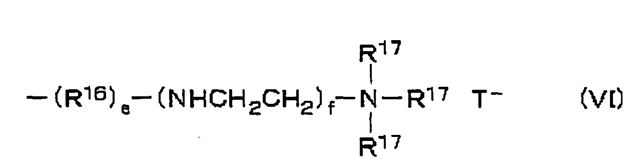

In the above general formulae (V) and (VI), R¹⁶ is a group represented by the following formulae:

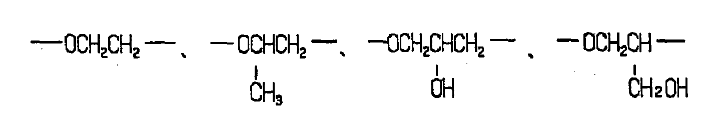

R¹⁷ and R¹⁸ are respectively a hydrogen atom or a monovalent hydrocarbon group, and may be the same or different from each other. In the above general formulae (V) and (VI), e and f are respectively an integer of 0 to 6, and T⁻ represents a halogen ion or an organic anion. Specific examples of T⁻ include halogen ions such as chloride ion, iodide ion and bromide ion; and organic anions such as a methosulfate anion, an ethosulfate anion, a methophosphate anion and an ethophosphate anion.

Examples of the preferred E group include -(CH₂)₃-NH₂, -(CH₂)₃-N(CH₃)₂, -(CH₂)₃-NH-(CH₂)₂-NH₂, -(CH₂)₂-NH-(CH₂)₂-N(CH₃)₂ and -(CH₂)₃N⁺-(CH₃)₃Cl⁻. Among these groups, more preferred is -(CH₂)₃-NH-(CH₂)₂-NH₂.

Z is a divalent carbon-containing organic group which is bonded to an adjacent silicon atom through a carbon-to-silicon bond and bonded to a polyoxyalkylene block chain through an oxygen atom. In particular, among these groups as Z, preferred are an alkylene group and an arylene group; more preferred are an ethylene group, a propylene group, a trimethylene group, a n-butylene group and an isobutylene group; and most preferred are a n-butylene group and an isobutylene group.

Also, in the above general formulae (V) and (VI), n is a number of 2 to 10 in which a plurality of n's in number of c may be the same or different from each other; a is a number of 2 or more and preferably from 2 to 1000; b is a number of 1 or more and preferably from 1 to 50 ; c is a number of 4 or more and preferably from 4 to 200; and d is a number of 2 or more and preferably from 2 to 100.

The content of the siloxane block in the block copolymer represented by the general formula (I) is preferably from 25 to 97% by mass, more preferably from 35 to 90% by mass and still more preferably from 50 to 80% by mass on the basis of a total amount of the block copolymer. The block copolymer preferably has an average molecular weight of at least 1200. Meanwhile, all of the average molecular weights used therein are measured by an ordinary GPC method using chloroform as an eluent and polystyrene as a standard substance.

Examples of the preferred amino-modified polysiloxane/polyoxyalkylene block copolymer include compounds represented by the following general formula (VII): 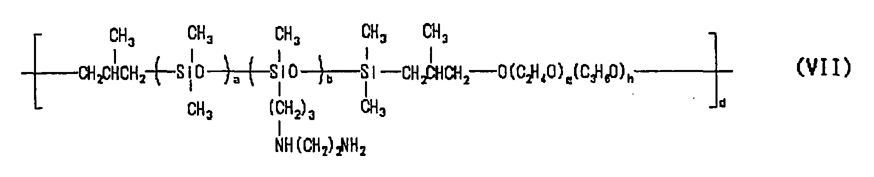 wherein a, b and d are respectively the same as defined above; g is a number of 4 or more; and h is a number of 0 to 30. In the general formula (VII), a is preferably a number of 2 to 1,000; b is preferably a number of 1 to 50; g is preferably a number of 4 to 200; and h is preferably a number of 2 to 100. Also, the segment of -O(C₂H₄O)_{g}(C₃H₆O)ₕ- may be in the form of either a block copolymer or a random copolymer. Further, the above copolymer may be produced, for example, by the method described in JP 9-183854A. In addition, examples of commercially available products of the copolymer include "FZ-3789" and "Silicone SS-3588" both available from Toray Dow Corning Silicone Co., Ltd.

The kinematic viscosity of the amino-modified silicone represented by the general formula (I) is preferably 10 mm²/s or higher, more preferably 100 mm²/s or higher, still more preferably 1,000 mm²/s or higher and most preferably 5,000 mm²/s or higher, and further preferably 1,000,000 mm²/s or lower and more preferably 100,000 mm²/s or lower. When the kinematic viscosity of the amino-modified silicone lies within the above-specified range, a residual amount of the oil agent phase (A) attached onto hair can be suitably enhanced. Meanwhile, the kinematic viscosity of the amino-modified silicone is measured at 25°C by a B-type viscometer using a rotor No. 2 at 6 rpm.

The amino-modified silicone represented by the general formula (I) has an amine equivalent of preferably 300 g/mol or higher and more preferably 600 g/mol or higher, and further preferably 10,000 g/mol or lower, more preferably 5,000 g/mol or lower and still more preferably 2,500 g/mol or lower. When the amine equivalent of the amino-modified silicone lies within the above-specified range, the hair treated with the resultant composition is reduced in frictional feel and improved in softness upon rinsing. Meanwhile, the amine equivalent (g/mol) of the amino-modified silicone is determined by subjecting an ethanol solution of the block copolymer to titration with a hydrochloric acid solution having a known concentration.

When the amino-modified silicone (amino-modified polydimethylpolysiloxane/polyoxyalkylene block copolymer) is previously uniformly mixed in the oil agent phase (A) together with the vegetable oil and the volatile silicone and then added to the gel emulsion, the resultant composition is enhanced in stability of the oil droplet particles therein and adsorption onto a surface of hair fibers, thereby attaining a more remarkable care effect. It is considered that the reason therefor is that when the copolymer is uniformly mixed in the oil agent phase, the surface of the respective oil droplet particles is electrified into a positive charge, thereby forming oil droplets having a good stability with the passage of time, and the resultant composition is readily adsorbed onto even damaged hydrophilized portions of hair.

When the amino-modified silicone is incorporated into the oil agent phase (A), the oil droplets of the oil agent phase (A) are further stabilized in the gel emulsion.

The content of the amino-modified silicone in the oil agent phase (A) is preferably in the range of 1 to 40% by mass. When the content of the amino-modified silicone lies within the above-specified range, the oil droplets of the oil agent phase (A) are further stabilized in the gel emulsion.

Further, the content of the amino-modified silicone in the hair cosmetic composition is from 0.01 to 10% by mass, preferably from 0.02 to 5% by mass and more preferably from 0.03 to 2% by mass in view of reducing a frictional feel of hair upon rinsing.

The oil droplet particles of the oil agent phase (A) preferably have a particle size of 1 to 20 µm. When the particle size of the oil droplet particles lies within the above-specified range, the obtained cosmetic composition can show an excellent feeling upon use and an excellent stability.

Further, the hair cosmetic composition of the present invention may also contain an oil agent phase (B) containing a non-volatile silicone in the form of oil droplet particles independently of the first oil droplet particles made of the oil agent phase (A). When the second oil droplet particles are present independently, the resultant composition imparts a dry smoothness, a softness and a luster after drying to hair and, therefore, exhibits an enhanced hair protecting effect. The presence of the two kinds of independent oil droplet particles can be confirmed by an imaging method using a co-focal Raman microscope. The imaging has been conducted at both wavelengths derived from the methyl group of the silicone (from 650 to 750 cm⁻¹) and alkyl group of the vegetable oil (from 950 to 1150 cm⁻¹).

The second oil droplet particles may coexist in gel emulsion independently of the first oil droplet particles by adding the oil agent phase containing the non-volatile silicone to the gel emulsion separately from the above oil agent phase (A) and then mixing the resultant mixture under stirring. The order of addition of the oil agent phases (A) and (B) is optional, and any of the oil agent phases (A) and (B) may be added first.

The non volatile silicone used in the oil agent phase (B) includes, for example, dimethyl polysiloxanes represented by the following general formula: 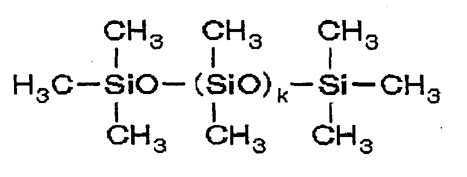 wherein k is an integer of 10 or more and preferably from 10 to 100000. The dimethyl polysiloxane having an especially high polymerization degree may be used upon blending in the form of a solution prepared by dissolving the dimethyl polysiloxane in a liquid oil (such as, for example, volatile silicones, low-viscous silicones and hydrocarbon oils) or in the form of an emulsion or a dispersion previously prepared in an aqueous solution of a cationic surfactant or a nonionic surfactant such as a polyoxyethylene alkyl ether. Examples of commercially available products of the dimethyl polysiloxane include "SH 200C" series, "10cs", "50cs", "200cs", "1000cs", "5000cs", "BY11-003", "BY11-026" and "BY22-019" all available from Toray Dow Corning Silicone Co., Ltd., and "KF-96" series, "1000cs", "5000cs", "KF9008" and "KF-9013" all available from Shin-Etsu Chemical Industry Co., Ltd.

Examples of the other non-volatile silicone include non-volatile silicone oils such as dimethyl cyclopolysiloxane, methyl phenyl polysiloxane, methyl hydrogen polysiloxane, octamethyl cyclotetrasiloxane, octamethyl cyclopentasiloxane and decamethyl cyclopentasiloxane.

Further, as the non-volatile silicone, there may also be used an amino-modified silicone represented by the following general formula as well as a fluorine-modified silicone, an alkyl-modified silicone, etc.: 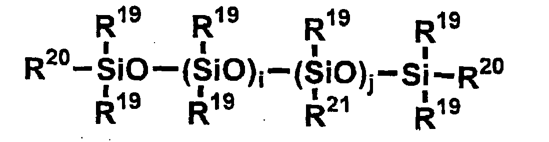 wherein R¹⁹ is a methyl group; R²⁰ is the same group as R²¹, or a methyl group or a hydroxyl group, and R²¹ is a reactive functional group represented by -R²²-Y wherein R²² is a divalent hydrocarbon group having 3 to 6 carbon atoms, and Y is a primary to tertiary amino-containing group or an ammonium-containing group; and i and j are respectively a positive integer and a sum of i and j (i + j) depend upon a molecular weight of the amino-modified silicone. The amino-modified silicone preferably has an average molecular weight of 3000 to 100000. Specific examples of the amino-modified silicone include commercially available products such as "SS-3551", "BY22-079 SF8452C", "DC8500" and "DC929" all available from Toray Dow Corning Silicone Co., Ltd., "KF-8004" available from Shin-Etsu Chemical Industry Co., Ltd., and "KT 1989" available from GE Toshiba Silicone Co., Ltd. Further, there may be used an aqueous emulsion containing the amino-modified silicone in an amount of 20 to 60% by mass and preferably 30 to 50% by mass such as, for example, "SM8904C" available from Toray Dow Corning Silicone Co., Ltd., etc.

The content of the non-volatile silicone as the oil agent phase (B) in the hair cosmetic composition of the present invention is preferably from 0.05 to 15% by mass, more preferably from 0.1 to 10% by mass and still more preferably from 0.1 to 5% by mass in view of imparting a good touch feel to hair. When the content of the oil agent phase (B) is 0.05% by mass or more, the resultant hair cosmetic composition fully exhibits the effects aimed by the present invention, whereas when the content of the oil agent phase (B) is 15% by mass or less, the resultant hair cosmetic composition is free from heavy feeling upon use such as stickiness at roots of hair fibers though it varies depending upon other components contained therein and composition of the gel emulsion.

The oil droplet particles of the oil agent phase (B) preferably have a particle size of 0.1 to 100 µm. When the particle size of the oil droplet particles of the oil agent phase (B) lies within the above-specified range, the obtained composition imparts a good feeling upon use, in particular, a good smoothness and a good slippery feel to hair.

The hair cosmetic composition of the present invention may also contain, in addition to the above components, optional components usable in ordinary hair cosmetics, if required. These optional components may be added at any of the below-mentioned steps according to properties of the respective components to be added. Examples of the optional components include hair mending components such as amino acids, amino acid derivatives and vitamins; anti-dandruff agents such as zinc pyrithion and benzalkonium chloride; extracts such as polar solvent extracts of eucalyptus, ginseng extracts, rice embryo extracts, fucus extracts, aloe extracts, alpinia leaf extracts and chlorella extracts; chelating agents; ultraviolet absorbers; antioxidants; antiseptic agents; colorants; and perfumes.

Examples of the amino acid include arginine, lysine, histidine, proline, cystine, methionine, serine, threonine, tyrosine, glutamine and isoleucine. Among these amino acids, especially preferred are arginine and lysine. Examples of the amino acid derivatives include trimethyl glycine, acylated amino acids, acylalkyl amino acids, and peptides such as dipeptides and tripeptides, as well as animal-derived proteins such as keratin, elastin, collagen, lactoferrin, casein, α(β)-lactalbumin, globulins and ovalbumin or hydrolyzed products thereof, and vegetable-derived proteins such as wheat, malt, soybean and silk proteins or hydrolyzed products thereof. Among these amino acid derivatives, preferred are keratin, elastin, collagen and casein and hydrolyzed products thereof, and wheat, soybean and silk proteins and hydrolyzed products thereof. Examples of the vitamins include tocopherol acetate, ascorbic acid, vitamin B1, vitamin B5, vitamin D, vitamin A, nicotinic acid amide, panthenol and panthenyl ethyl ether. Among these vitamins, preferred are tocopherol acetate, panthenol and panthenyl ethyl ether.

The hair cosmetic composition of the present invention has a pH of 1 to 7 in view of usability without problems. In particular, in view of enhancing luster and manageability of hair, the pH of the hair cosmetic composition used is preferably in a lower range. The pH of a dilute solution prepared by diluting the composition with water to 1/20 time by weight, is preferably in the range of 1.5 to 5.0, more preferably 2.5 to 4.5 and still more preferably 2.8 to 4.2 as measured at 25°C. The pH of the hair cosmetic composition may be controlled by using the above acid components or other acid components as well as inorganic or organic basic substances such as sodium hydroxide and organic amines.

The hair cosmetic composition of the present invention may be produced by the process including a first step of mixing an oil phase containing at least one fatty alcohol with a cationic surfactant and an aqueous carrier to form a gel emulsion; and a second step of adding and mixing an oil agent phase (A) separately prepared by mixing a vegetable oil and a volatile silicone with each other in the gel emulsion under stirring to form oil droplet particles therein.

Also, when the hair cosmetic composition further contains oil droplet particles made of an oil agent phase (B) containing a non-volatile silicone, the hair cosmetic composition may be produced by independently adding, after the first step, the oil agent phase (A) and the oil agent phase (B) separately prepared by mixing the respective components to the gel emulsion, and mixing the obtained composition under stirring to allow the oil agent phase (A) and the oil agent phase (B) to coexist in the form of oil droplet particles in the gel emulsion.

In the production process of the present invention, the order of addition of the oil agent phase (A) and the oil agent phase (B) to the gel emulsion is optional, and any of the oil agent phase (A) and the oil agent phase (B) may be added first to the gel emulsion. Also, after adding any one of the oil agent phase (A) and the oil agent phase (B) to the gel emulsion and mixing the mixture under stirring, the other phase may be added to the mixture, followed by mixing the obtained composition under stirring.

Further, the oil agent phase (A) and the oil agent phase (B) may be added to the gel emulsion at the same time. In addition, when the oil agent phase (B) is in the form of a dispersion (emulsion), after adding the oil agent phase (A) to the oil agent phase (B), the resultant mixed phase may be added to the gel emulsion, followed by mixing the obtained composition under stirring.

The hair cosmetic composition of the present invention is preferably used as products such as a hair rinse, a hair conditioner, a hair treatment, a hair pack and a hair cream. Further, the hair cosmetic composition produced through the above first and second steps may be used in a product configuration such as a conditioning mousse (conditioning foam), a hair mousse (hair foam), a hair spray, a shampoo, a leave-on-treatment, a wax, a tonic and a hair lotion, as well as a hair color in the form of a mixture with dyes, a reforming agent etc.

### EXAMPLES

### Evaluation of Hair Treated

Evaluation of hair treated with the hair cosmetic composition was made by 10 expert panelists as follows. That is, hair of each panelist was washed with a commercially available shampoo, and then treated with the respective hair cosmetic compositions obtained in Examples and Comparative Examples, followed by rinsing the hair. This procedure was repeated one time every day for two weeks.

The hair cosmetic compositions used above were evaluated for spreadability upon application and smoothness upon rinsing as well as lack of stickiness, lack of dry and rough feel, moistened feel, smoothness and luster after drying on the basis of the following evaluation criteria.
A: Effectiveness of the composition was confirmed by 8 or more persons;
B: Effectiveness of the composition was confirmed by 6 to 7 persons;
C: Effectiveness of the composition was confirmed by 4 to 5 persons;
D: Effectiveness of the composition was confirmed only by 3 or less persons.

### EXAMPLE 1

An oil phase composed of stearyl alcohol and stearoxypropyldimethylamine (75°C) was added to an aqueous phase containing lactic acid (60°C) under stirring to prepare a gel emulsion (first step). Then, an oil agent phase (A) separately prepared by mixing dodecamethyl cyclopentasiloxane and a high-oleic content sunflower oil with each other was added to the gel emulsion, and the resultant mixture was mixed under stirring to form oil droplet particles made of the oil agent phase (A) in the gel emulsion (second step), thereby obtaining a hair cosmetic composition. The thus obtained hair cosmetic composition was evaluated by the above method. The results are shown in Table 1.

### EXAMPLE 2

The same procedure as in Example 1 was repeated except that an amino-modified silicone was added and mixed in the gel emulsion upon mixing the oil agent phase (A) therein, and after forming the oil droplet particles, the remaining components were added to the gel emulsion, thereby obtaining a hair cosmetic composition. The thus obtained hair cosmetic composition was evaluated by the above method. The results are shown in Table 1.

### EXAMPLE 3

The same procedure as in Example 1 was repeated except that dipropylene glycol was further added in the first step, an amino-modified silicone was added and mixed in the gel emulsion upon mixing the oil agent phase (A) therein, and after forming the oil droplet particles, the remaining components were added to the gel emulsion, thereby obtaining a hair cosmetic composition. The thus obtained hair cosmetic composition was evaluated by the above method. The results are shown in Table 1.

### EXAMPLES 4 TO 10

After completing the first step but before forming the oil droplet particles made of the oil agent phase (A) in the second step, an oil agent phase (B) was further added to the gel emulsion and mixed therewith under stirring to form second oil droplet particles in the gel emulsion. Next, components forming the oil agent phase (A) were added to the gel emulsion and mixed therewith under stirring to form the oil droplet particles made of the oil agent phase (A) in the gel emulsion, thereby obtaining a hair cosmetic composition. The thus obtained hair cosmetic composition was evaluated by the above method. The results are shown in Table 1.

### COMPARATIVE EXAMPLES 1 AND 2

The same procedure as in Example 1 was repeated except that a vegetable oil was melted and mixed in the fatty alcohol, the cationic surfactant, etc., in the first step to prepare a gel emulsion, thereby obtaining a hair cosmetic composition. The thus obtained hair cosmetic composition was evaluated by the above method. The results are shown in Table 1.

**TABLE 1-1**

| | Examples | | | |
|---|---|---|---|---|
| | 1 | 2 | 3 | 4 |
| Oil agent phase (A) | | | | |
| Decamethyl cyclopentasiloxane^{*1} | 0.8 | 0.8 | 0.8 | 0.8 |
| Dodecamethyl pentasiloxane^{*2} | - | - | - | - |
| High-oleic content sunflower oil | 0.2 | 0.2 | 0.2 | 0.2 |
| Tsubaki oil | - | - | - | - |
| Olive oil | - | - | - | - |
| Avocado oil | - | - | - | - |
| Amino-modified polydimethylpolysiloxane/polyoxyalkylene block copolymer ^{*3} | - | 0.1 | 0.1 | 0.1 |
| Oil agent phase (B) | | | | |
| Dimethyl polysiloxane^{*4} | - | - | - | 2.5 |
| Constituents of gel emulsion | | | | |
| Stearoxypropyldimethylamine | 1.5 | 1.5 | 1.5 | 1.5 |
| Stearamidopropyl dimethylamine | - | - | - | - |
| Behenamidopropyl dimethylamine | - | - | - | - |
| Behenyltrimethylammonium chloride | - | - | - | - |
| Cetyl alcohol | - | - | - | - |
| Stearyl alcohol | 5.5 | 5.5 | 5.5 | 5.5 |
| Behenyl alcohol | - | - | - | - |
| Dipropylene glycol | - | - | 2.5 | 2.5 |
| Benzyl alcohol | - | 0.3 | 0.3 | 0.3 |
| Lactic acid (90%) (1) [for forming a gel matrix] | 0.4 | 0.4 | 0.4 | 0.4 |
| Lactic acid (90%) (2) [for adjusting pH] | - | q.s. | q.s. | q.s. |
| Water | Balance | Balance | Balance | Balance |
| Viscosity | 8000 | 9000 | 8500 | 10000 |
| pH (upon diluting with water to 1/20 time) | 4.5 | 3.2 | 3.2 | 3.2 |
| Sensory evaluation | | | | |
| Spreadability upon application | B | B | A | A |
| Smoothness upon rinsing | A | A | A | A |
| Lack of stickiness after drying | A | A | A | A |
| Lack of dry and rough feel after drying | B | B | B | A |
| Moistened feel after drying | B | B | B | A |
| Smoothness after drying | B | B | B | A |
| Luster after drying | B | A | A | A |

**TABLE 1-2**

| | Examples | | | |
|---|---|---|---|---|
| | 5 | 6 | 7 | 8 |
| Oil agent phase (A) | | | | |
| Decamethyl cyclopentasiloxane^{*1} | 0.8 | 0.8 | 0.8 | - |
| Dodecamethyl pentasiloxane^{*2} | - | - | - | 0.8 |
| High-oleic content sunflower oil | - | - | - | 0.2 |
| Tsubaki oil | 0.2 | - | - | - |
| Olive oil | - | 0.2 | - | - |
| Avocado oil | - | - | 0.2 | - |
| Amino-modified polydimethylpolysiloxane/polyoxyalkylene block copolymer^{*3} | 0.1 | 0.1 | 0.1 | 0.1 |
| Oil agent phase (B) | | | | |
| Dimethyl polysiloxane^{*4} | 2.5 | 2.5 | 2.5 | 2.5 |
| Constituents of gel emulsion | | | | |
| Stearoxypropyldimethylamine | 1.5 | 1.5 | 1.5 | 1.5 |
| Stearamidoproryl dimethylamine | - | - | - | - |
| Behenamidopropyl dimethylamine | - | - | - | - |
| Behenyltrimethylammonium chloride | - | - | - | - |
| Cetyl alcohol | - | - | - | - |
| Stearyl alcohol | 5.5 | 5.5 | 5.5 | 5.5 |
| Behenyl alcohol | - | - | - | - |
| Dipropylene glycol | 2.5 | 2.5 | 2.5 | 2.5 |
| Benzyl alcohol | 0.3 | 0.3 | 0.3 | 0.3 |
| Lactic acid (90%) (1) [for forming a gel matrix] | 0.4 | 0.4 | 0.4 | 0.4 |
| Lactic acid (90%) (2) [for adjusting pH] | q.s. | q.s. | q.s. | q.s. |
| Water | Balance | Balance | Balance | Balance |
| Viscosity | 9500 | 11000 | 10000 | 8000 |
| pH (upon diluting with water to 1/20 time) | 3.2 | 3.2 | 3.2 | 3.2 |
| Sensory evaluation | | | | |
| Spreadability upon application | A | A | A | A |
| Smoothness upon rinsing | A | A | A | A |
| Lack of stickiness after drying | A | A | A | A |
| Lack of dry and rough feel after drying | A | A | A | A |
| Moistened feel after drying | A | A | A | A |
| Smoothness after drying | A | A | A | A |
| Luster after drying | A | A | A | A |

**TABLE 1-3**

| | Examples | | Comparative Examples | |
|---|---|---|---|---|
| | 9 | 10 | 1 | 2 |
| Oil agent phase (A) | | | | |
| Decamethyl cyclopentasiloxane^{*1} | 0.8 | 0.8 | 0.8 | 0.8 |
| Dodecamethyl pentasiloxane^{*2} | - | - | - | - |
| High-oleic content sunflower oil | 0.2 | 0.2 | 0.2 | 0.2 |
| Tsubaki oil | 0.1 | - | - | - |
| Olive oil | - | 0.05 | - | - |
| Avocado oil | - | 0.05 | - | - |
| Amino-modified polydimethylpolysiloxane/polyoxyalkylene block copolymer^{*3} | 0.1 | 0.1 | - | 0.1 |
| Oil agent phase (B) | | | | |
| Dimethyl polysiloxane^{*4} | 2.5 | 2.5 | - | - |
| Constituents of gel emulsion | | | | |
| Stearoxypropyldimethylamine | - | 1 | 1.5 | 1.5 |
| Stearamidopropyl dimethylamine | 0.8 - | - | - | - |
| Behenamidopropyl dimethylamine | 0.9 | - | - | - |
| Behenyltrimethylammonium chloride | - | 0.6 | - | - |
| Cetyl alcohol | 1 | 0.5 | - | - |
| Stearyl alcohol | 4 | 5 | 5.5 | 5.5 |
| Behenyl alcohol | 0.5 | - | - | - |
| Dipropylene glycol | 2.5 | 2.5 | - | - |
| Benzyl alcohol | 0.3 | 0.3 | - | 0.3 |
| Lactic acid (90%) (1) [for forming a gel matrix] | 0.4 | 0.4 | 0.4 | 0.4 |
| Lactic acid (90%) (2) [for adjusting pH] | q.s. | q.s. | - | q.s. |
| Water | Balance | Balance | Balance | Balance |
| Viscosity | 8500 | 11000 | 8500 | 9500 |
| pH (upon diluting with water to 1/20 time) | 3.2 | 3.2 | 4.5 | 3.2 |
| Sensory evaluation | | | | |
| Spreadability upon application | A | A | B | B |
| Smoothness upon rinsing | B | B | C | B |
| Lack of stickiness after drying | A | A | B | B |
| Lack of dry and rough feel after drying | A | A | B | B |
| Moistened feel after drying | A | A | B | B |
| Smoothness after drying | A | A | B | B |
| Luster after drying | A | A | B | A |

| | | | | |
|---|---|---|---|---|
| Note: *1: "SH245 Fluid" available from Toray Dow Corning Silicone Co., Ltd. *2: "SH200 C Fluid" available from Toray Dow Corning Silicone Co., Ltd. *3: "SS-3588" available from Toray Dow Corning Silicone Co., Ltd. *4: "BY22-060" available from Toray Dow Corning Silicone Co., Ltd. | | | | |

### EXAMPLE 11

### <Composition of Conditioner (pH: 3.3)>

| | |
|---|---|
| Stearoxypropyldimethylamine | 1.7% |
| Stearyl alcohol | 5.8% |
| High-oleic content sunflower oil | 0.2% |
| Silicone (SH245)^{*1} | 0.5% |
| Lactic acid | 1.6% |
| Glycolic acid | 0.2% |
| N,N'-bis(3-methoxypropyl)isodocosane diamide | 0.1% |
| Benzyl alcohol | 0.5% |
| Dipropylene glycol | 2.5% |
| Silicone (BY11-003)^{*5} | 1.0% |
| Silicone (KHS-3)^{*6} | 2.0% |
| Amino-modified silicone (SM8704C)^{*7} | 0.5% |
| Amino-modified silicone-polyoxyalkylene block copolymer^{*8} | 0.2% |
| Hydroxyethyl cellulose (SE-850K)^{*9} | 0.3% |
| Highly-polymerized polyethylene glycol | 0.1% |
| Extract of prune | 0.02% |
| Perfume | q.s. |
| Purified water | Balance |

| | |
|---|---|
| Note: *1: Available from Toray Dow Corning Silicone Co., Ltd. *5: Available from Toray Dow Corning Silicone Co., Ltd. *6: Available from Shin-Etsu Chemical Industry Co., Ltd. *7: Available from Toray Dow Corning Silicone Co., Ltd. *8: Available from Toray Dow Corning Silicone Co., Ltd. *9: Available from Daicel Chemical Industry Co., Ltd. | |

### EXAMPLE 12

### <Composition of Conditioner (pH: 3.5)>

| | |
|---|---|
| Stearoxypropyldimethylamine | 1.5% |
| Behenamide propyldimethylamine | 0.5% |
| Stearyl alcohol | 4.0% |
| Cetyl alcohol | 2.0% |
| Tsubaki oil | 0.2% |
| Silicone (SH245)^{*1} | 0.5% |
| Dipentaerythritol fatty ester (COSMOL 168AR)^{*10} | 0.2% |
| Lactic acid (90%) | 1.5% |
| Malic acid (50%) | 0.1% |
| Benzyloxyethanol | 0.5% |
| Dipropylene glycol | 2.5% |
| Silicone (KHS-3)^{*6} | 2.5% |
| Amino-modified silicone (SM8704C)^{*7} | 1.0% |
| Amino-modified silicone-polyoxyalkylene block copolymer^{*8} | 0.2% |
| Hydroxyethyl cellulose (SE-850K)^{*9} | 0.3% |
| Extract of eucalyptus | 0.02% |
| Extract of soybean | 0.02% |
| Caramel | 0.03% |
| Perfume | q.s. |
| Purified water | Balance |

| | |
|---|---|
| Note: *1: Available from Toray Dow Corning Silicone Co., Ltd. *6: Available from Shin-Etsu Chemical Industry Co., Ltd. *7: Available from Toray Dow Corning Silicone Co., Ltd. *8: Available from Toray Dow Corning Silicone Co., Ltd. *9: Available from Daicel Chemical Industry Co., Ltd. *10: Available from Nissin Oilio Group Co., Ltd. | |

The hair conditioners obtained in Examples 11 and 12 were prevented from suffering from greasy feel and sticky feel upon application to hair, and were excellent in softness and smoothness upon application and rinsing as well as soft feel and comb-passing feel after drying.

## Claims

1. A hair cosmetic composition comprising a gel emulsion containing at least one fatty alcohol, a cationic surfactant and an aqueous carrier, and (A) an oil agent phase containing a vegetable oil and a volatile silicone, wherein the oil agent phase (A) is present in the form of oil droplet particles in the gel emulsion.

2. The hair cosmetic composition according to claim 1, wherein the volatile silicone is a cyclic silicone.

3. The hair cosmetic composition according to claim 1 or 2, wherein the oil agent phase (A) further contains an amino-modified silicone represented by the following general formula (I): 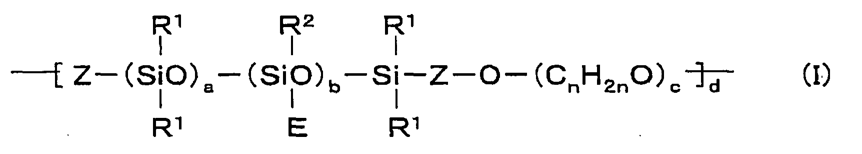 wherein R¹ is a hydrogen atom or a monovalent hydrocarbon group having 1 to 6 carbon atoms; R² is either R¹ or E; E is a group represented by the formula: -R³-W wherein R³ is a direct bond or a divalent hydrocarbon group having 1 to 20 carbon atoms, and W is a primary to tertiary amino-containing group or an ammonium-containing group; Z is a divalent carbon-containing organic group which is bonded to an adjacent silicon atom through a carbon-to-silicon bond and bonded to a polyoxyalkylene block chain through an oxygen atom; n is a number of 2 to 10 in which a plurality of n's in number of c may be the same or different from each other; a is a number of 2 or more; b is a number of 1 or more; c is a number of 4 or more; and d is a number of 2 or more.

4. The hair cosmetic composition according to any one of claims 1 to 3, wherein the gel emulsion further contains (B) an oil agent phase containing a non-volatile silicone, and the oil droplet particles of the oil agent phase (A) and oil droplet particles of the oil agent phase (B) coexist in the gel emulsion.

5. A process for producing a hair cosmetic composition, comprising the steps of:
mixing at least one fatty alcohol, a cationic surfactant and an aqueous carrier with each other to prepare a gel emulsion; and
adding (A) an oil agent phase containing a vegetable oil and a volatile silicone to the gel emulsion to allow the oil agent phase (A) to be present in the form of oil droplet particles in the gel emulsion.

6. The process according to claim 5, further comprising the step of adding to the gel emulsion upon mixing the oil agent phase (A) in the gel emulsion, an amino-modified silicone represented by the following general formula (I): 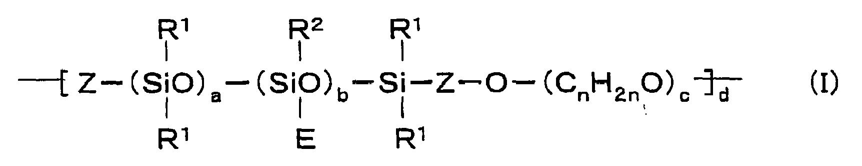 wherein R¹ is a hydrogen atom or a monovalent hydrocarbon group having 1 to 6 carbon atoms; R² is either R¹ or E; E is a group represented by the formula : -R³-W wherein R³ is a direct bond or a divalent hydrocarbon group having 1 to 20 carbon atoms, and W is a primary to tertiary amino-containing group or an ammonium-containing group; Z is a divalent carbon-containing organic group which is bonded to an adjacent silicon atom through a carbon-to-silicon bond and bonded to a polyoxyalkylene block chain through an oxygen atom; n is a number of 2 to 10 in which a plurality of n's in number of c may be the same or different from each other; a is a number of 2 or more; b is a number of 1 or more; c is a number of 4 or more; and d is a number of 2 or more.

7. The process according to claim 5 or 6, further comprising the step of adding (B) an oil agent phase containing a non-volatile silicone to the gel emulsion to allow the oil agent phase (A) and the oil agent phase (B) to be respectively independently present in the form of oil droplet particles.
